# FASCICULE DE BREVET EUROPEEN

(11) **EP 1 071 477 B1**
(45) Date de publication et mention de la délivrance du brevet: **26.03.2003**
(21) Numéro de dépôt: 99913395.2
(22) Date de dépôt: 13.04.1999
(51) Int. Cl.: A61L 2/18

(54) **DISPOSITIF DE CONDITIONNEMENT ET DE TRAITEMENT BACTERICIDE POUR LENTILLES DE CONTACT**
VORRICHTUNG ZUR KONDITIONIERUNG UND BAKTERIZIDEN BEHANDLUNG VON KONTAKTLINSEN
DEVICE FOR PACKAGING AND TREATING BACTERICIDE FOR CONTACT LENSES

(30) Priorité: 14.04.1998 FR 9804612
(43) Date de publication de la demande: 31.01.2001
(73) Titulaire: Rexam Dispensing Systems, 76470 Le Tréport (FR)
(72) Inventeur: BOUGAMONT, Jean-Louis, F-76260 Eu (FR); HENNEMANN, Pascal, F-76260 Eu (FR); LEULIET, David, F-80350 Mers-les-Bains (FR)
(74) Mandataire: Busnel, Jean-Benoît
(86) Numéro de dépôt international: FR9900858
(87) Numéro de publication internationale: WO99052568

(56) Documents cités:
- EP-A- 0 049 767
- EP-A- 0 830 865
- WO-A-95/26756
- WO-A-96/36371

## Description

La présente invention concerne un dispositif de conditionnement et de traitement bactéricide pour lentilles de contact.

Les lentilles cornéennes ou de contact doivent impérativement être protégées de toute contamination bactérienne ou bien être décontaminées avant d'être mises en place afin d'éviter toute infection oculaire.

Cest la raison pour laquelle, il existe des dispositifs permettant de plonger les lentilles dans une solution de traitement bactéricide. Ces dispositifs comprennent notamment des moyens de suspension desdites lentilles associés généralement à des moyens d'obturation du réservoir de solution bactéricide.

Cependant, ces dispositifs sont peu pratiques.

En effet, le transport du réservoir de liquide de traitement est une opération délicate et ceci d'autant plus que ce réservoir est généralement de petites dimensions.

Au surplus, en cas d'erreur de manipulation, les lentilles tombent dans le fond du réservoir et sont difficiles à récupérer.

De plus, le mauvais verrouillage des moyens d'obturation peut entraîner une rupture de l'étanchéité du réservoir préjudiciable tant à la protection des lentilles qu'à l'utilisateur, du fait de l'apparition de fuites de liquide bactéricides.

En outre, après immersion, les lentilles qui possèdent une certaine courbure retiennent parfois une fraction très faible de solution bactéricide.

Or, les solutions bactéricides contiennent généralement un agent conservateur qui provoque des effets secondaires indésirables sur l'organisme.

Certains de ces problèmes ont déjà été résolus dans l'état de la technique. Ainsi, le document EP-A-0 830 865 décrit un dispositif pour le traitement de lentilles de contact par simple contact des lentilles avec des surfaces germicides convexe-concave, adaptées à la courbure de la lentille. En particulier, ce document décrit un dispositif avec une cavité, dans laquelle est placé un élément de support germicide et un élément de surface germicide lui aussi. La lentille est coincée entre les deux éléments.

La présente invention a pour but de permettre un positionnement précis et une protection accrue des lentilles.

Ce but est atteint conformément à l'invention, au moyen d'un dispositif selon la revendication 1.

Selon une caractéristique avantageuse, lesdits éléments de support et de calage sont réalisés en une matière poreuse contenant un agent antibactérien non migrant.

Selon une autre caractéristique, l'élément de calage est en serrage radial dans la douille.

Selon encore une autre caractéristique, le bord de ladite cavité est pourvu d'une collerette dont la paroi intérieure possède un chanfrein destiné à assurer le guidage de la douille.

De même, le bord périphérique de la douille possède un chanfrein de guidage.

De préférence, le fond de la cavité comporte un alésage de diamètre restreint dans lequel l'élément de support est en serrage radial.

Il est prévu que la face externe de l'élément de support est concave et que la face externe de l'élément de calage soit convexe et fasse saillie au-delà du bord périphérique de la douille.

Selon un mode de réalisation spécifique, les éléments de support et de calage sont des cylindres amovibles de même diamètre.

Les dimensions respectives de la cavité et de la douille sont déterminées de telle sorte que la paroi externe de ladite douille vienne en contact coulissant avec la paroi interne de ladite cavité.

Selon un mode de réalisation particulier, le boîtier comprend deux cavités.

Selon une première variante de réalisation, le dispositif comprend un seul couvercle, pourvu de deux douilles destinées à s'introduire dans lesdites cavités, et assemblé sur le boîtier par emmanchement avec serrage radial périphérique en formant un cylindre parfait.

Selon une seconde variante, le dispositif comprend deux couvercles (2a,2b) pourvus chacun d'une douille (20a,20b).

Afin de renforcer la protection biologique, le boîtier et le ou les couvercles peuvent être réalisés avec une matière plastique contenant un agent antibactérien non migrant.

Le dispositif de l'invention a une structure simple et est facile à utiliser en améliorant notamment la dépose et la reprise des lentilles. Il offre toute sécurité pour le stockage et le transport des lentilles même en cas de chocs, de retournement, ou de renversement du boîtier.

Ce dispositif assure donc une protection mécanique et bactérienne des lentilles avec un degré élevé d'efficacité.

Au surplus, ce dispositif offre un emballage particulièrement ergonomique et esthétique.

L'invention sera mieux comprise à la lecture de la description qui va suivre accompagnée des dessins sur lesquels :
- les figures 1a et 1b représentent des vues d'un premier mode de réalisation du dispositif de l'invention, respectivement en perspective éclatée et en coupe transversale ;
- les figures 2a et 2b représentent des vues d'un second mode de réalisation du dispositif de l'invention respectivement en perspective éclatée et en coupe transversale.

Le dispositif représenté sur les figures est destiné au conditionnement et au traitement bactéricide de lentilles de contact L.

Ce dispositif comprend un boîtier 1 pourvu d'au moins une cavité 10 et ici de deux cavités 10a,10b.

Dans chaque cavité 10 est logé un élément de support 11 d'une lentille L dont la face supérieure 11a possède une courbure adaptée à celle desdites lentilles.

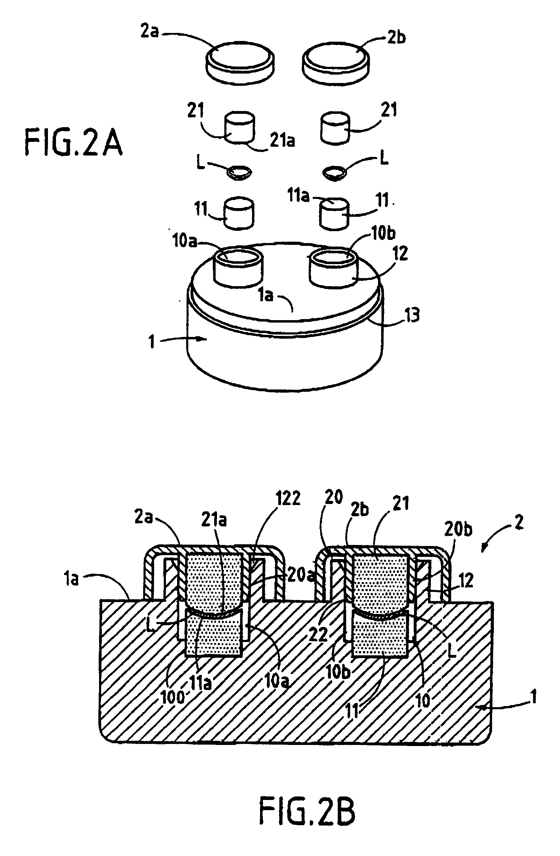

Lorsque le boîtier 1 est posé sur un plan horizontal, les lentilles L reposent sur la face supérieure, de préférence concave de l'élément de support 11 comme représenté sur la figure 2.

Le dispositif comprend au moins un couvercle 2 destiné à recouvrir les cavités 10a,10b.

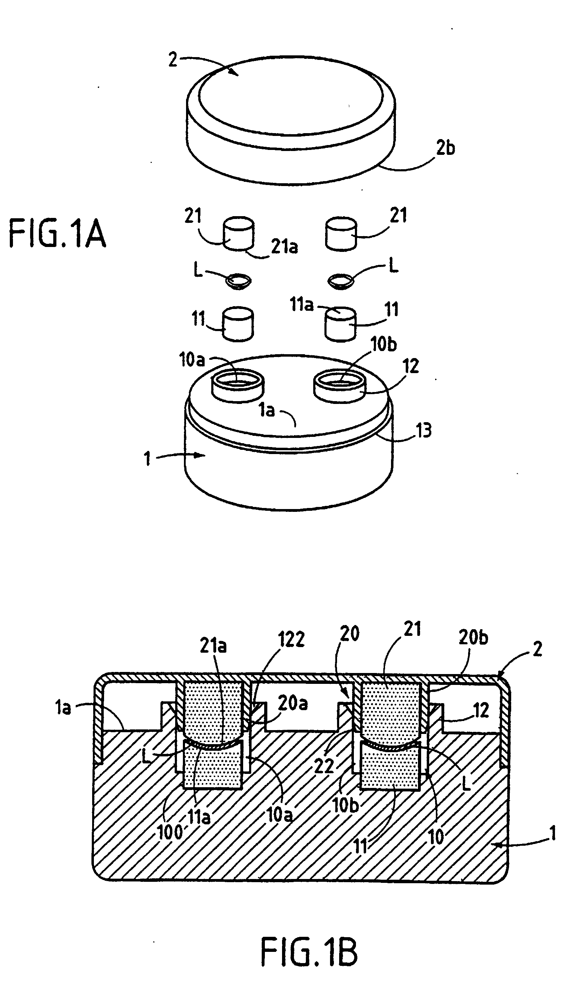

Dans le mode de réalisation des figures 1a et 1b, le couvercle est unique et recouvre simultanément les deux cavités.

Dans le mode de réalisation des figures 2a et 2b, chaque cavité 10a, 10b est recouverte par un couvercle 2a,2b indépendant.

Sur les figures 1a et 1b, la face interne du couvercle 2 est pourvue d'au moins une douille 20 et ici de deux douilles 20a,20b susceptibles d'être engagées de façon guidée dans les cavités respectives 10a,10b du boîtier 1.

Dans chacune des douilles 20a,20b est logé un élément de calage 21 dont la face inférieure externe 21a possède une courbure complémentaire de celle de l'élément de support 11 qui est donc ici convexe.

De manière générale, le nombre, la position et les dimensions des douilles 20 sont adaptés aux cavités 10.

Les éléments de support 11 et de calage 21 sont réalisés avec une matière plastique de préférence poreuse ayant une action bactéricide.

Cette matière peut être une matière plastique contenant un agent antibactérien non migrant, par exemple à base d'argent, qui assure le traitement bactéricide et plus précisément la décontamination bactérienne par simple contact avec la face en regard de la lentille L.

De préférence, le boîtier 1, le couvercle 2 ou les couvercles 2a,2b sont également réalisés, par exemple par moulage, avec une matière plastique contenant un agent antibactérien non migrant.

Cette disposition permet d'assurer la protection et/ou la décontamination des lentilles L en immobilisant chacune d'entre elles à l'intérieur d'une cavité 10, entre un élément de support 11 et un élément de calage 21 quelle que soit l'orientation du dispositif dans l'espace.

Les éléments de support 11 et de calage 21 sont des cylindres de même diamètre. Le fond de la cavité 10 comporte un alésage 100 de diamètre restreint dans lequel est fixé l'élément de support 11 par un serrage radial accompagné d'une légère compression élastique.

Le bord de la cavité 10 est pourvu d'une collerette 12 qui fait saillie sur la face supérieure 1a du boîtier 1.

La paroi interne de la collerette 12 et en particulier son rebord supérieur possède un chanfrein tronconique 12a destiné à faciliter l'introduction de la douille 20 dans la cavité 10 en assurant son guidage.

La hauteur de l'élément de support 11 est déterminée en fonction de la hauteur de la collerette 12 et des profondeurs respectives de la cavité 10 et de l'alésage 100 pour que sa face externe concave 11a se situe sous le niveau de la face supérieure 1a du boîtier 1.

L'élément de calage 21 est également fixé dans la douille 20 de manière éventuellement amovible, par un serrage radial accompagné d'une compression élastique.

La face externe 21a de l'élément de calage 21 fait saillie au-delà du bord périphérique de la douille 20 de façon à ce que le bord de la douille ne vienne pas toucher la lentille L.

De préférence, la face externe de l'élément de support 11 est concave pour stabiliser la lentille L tandis que la face externe de l'élément de calage 21 est convexe pour assurer un léger appui sur la lentille permettant d'éviter ainsi tout déplacement.

Le bord périphérique de la douille 20 possède un chanfrein tronconique coopérant avec le chanfrein 122 de la collerette 12 du boîtier 1 dont le profil est complémentaire en vue de guider la douille 20 dans la collerette 12 par contact de glissement.

Les dimensions respectives de la cavité 10 et de la douille 20 sont déterminées pour que lors de la mise en place du couvercle 2 sur le boîtier 1, la paroi latérale externe de la douille 20 vienne en contact coulissant avec la paroi interne de la cavité 10

Dans le mode de réalisation des figures 1a,1b, le boîtier 1 et le couvercle 2 sont réalisés sous forme indépendante et sont assemblés par emmanchement avec serrage radial périphérique en formant un cylindre parfait.

A cet effet, le boîtier 1 comporte un épaulement 13d destiné à recevoir de manière affleurante le bord inférieur de la paroi latérale du couvercle 2.

Dans le mode de réalisation des figures 2a,2b chaque couvercle 2a,2b est pourvu d'une douille 20a,20b et le bord inférieur de la paroi latérale des couvercles vient en appui sur la face supérieure 1a du boîtier.

Selon une variante non représentée, le couvercle 2 est articulé sur le boîtier 1 par exemple au moyen d'une charnière. Cette variante peut s'appliquer égs4alement au mode de réalisation à deux couvercles des figures 2a,2b en fixant la charnière sur la face supérieure du boîtier.

L'extraction des lentilles L à partir du dispositif s'effectue, par exemple, en renversant le boîtier 1 qui se trouve alors sur le dessus du dispositif et en le séparant du couvercle 2 qui se trouve quant à lui en dessous sur un plan horizontal.

Comme la face concave 21a de l'élément de calage 21 fait saillie à l'extérieur de la douille 20, la lentille L est alors facilement accessible pour l'utilisateur.

## Revendications

1. Dispositif de conditionnement et de traitement bactéricide pour lentilles (L) de contact, comprenant, d'une part,
- un boîtier (1) pourvu d'au moins une cavité (10) dans laquelle est logé un élément de support (11) à action bactéricide dont la face externe (11a) possède une courbure adaptée à celle des lentilles (L) et, d'autre part,
- au moins un couvercle (2) destiné à recouvrir ladite cavité (10) comprenant un élément de calage (21) à action bactéricide dont la face externe (21a) possède une courbure complémentaire de celle de l'élément de support (11) de façon à assurer la protection et/ou la décontamination d'une lentille (L) en l'immobilisant dans ladite cavité (10) entre ledit élément de support (11) et ledit élément de calage (21), **caractérisé en ce que** la face interne de la cavité (10) est pourvue d'au moins une douille (20) susceptible d'être engagée de façon guidée dans ladite cavité (10), l'élément de calage (21) étant logé dans la douille (20).

2. Dispositif selon la revendication 1, **caractérisé en ce que** lesdits éléments de support (11) et de calage (21) sont réalisés en une matière poreuse contenant un agent antibactérien non migrant.

3. Dispositif selon l'une des revendications précédentes, **caractérisé en ce que** l'élément de calage (21) est en serrage radial dans la douille (20).

4. Dispositif selon l'une des revendications précédentes, **caractérisé en ce que** le bord de ladite cavité (10) est pourvu d'une collerette (12) dont la paroi intérieure possède un chanfrein (122) destiné à assurer le guidage de la douille (20).

5. Dispositif selon l'une des revendications précédentes, **caractérisé en ce que** le fond de la cavité (10) comporte un alésage (100) de diamètre restreint dans lequel l'élément de support (11) est en serrage radial.

6. Dispositif selon l'une des revendications précédentes, **caractérisé en ce que** le bord périphérique de la douille (20) possède un chanfrein (22) de guidage.

7. Dispositif selon l'une des revendications précédentes, **caractérisé en ce que** la face externe (11a) de l'élément de support (11) est concave.

8. Dispositif selon l'une des revendications précédentes, **caractérisé en ce que** la face externe (21a) de l'élément de calage (21) fait saillie au-delà du bord périphérique de la douille (20).

9. Dispositif selon l'une des revendications précédentes, **caractérisé en ce que** les éléments de support (11) et de calage (21) sont des cylindres amovibles de même diamètre.

10. Dispositif selon l'une des revendications précédentes, **caractérisé en ce que** les dimensions respectives de la cavité (10) et de la douille (20) sont déterminées de telle sorte que la paroi externe de ladite douille vienne en contact coulissant avec la paroi interne de ladite cavité.

11. Dispositif selon l'une des revendications précédentes, **caractérisé en ce que** le boîtier (1) comprend deux cavités (10a,10b).

12. Dispositif selon la revendication 11, **caractérisé en ce qu'**il comprend un seul couvercle (2),pourvu de deux douilles (20a,20b) destinées à s'introduire dans lesdites cavités, et assemblé sur le boîtier (1) emmanchement avec serrage radial périphérique en formant un cylindre parfait.

13. Dispositif selon la revendication 11, **caractérisé en ce qu'**il comprend deux couvercles (2a,2b) pourvus chacun d'une douille (20a,20b).

14. Dispositif selon l'une des revendications précédentes, **caractérisé en ce que** le boîtier (1) et le couvercle (2) sont réalisés avec une matière plastique contenant un agent antibactérien non migrant.

## Claims

1. A packaging and bactericidal treatment device for contact lenses (L), comprising:
firstly a housing (1) provided with at least one cavity (10) receiving a support element (11) which has bactericidal action and whose outside face (11a) has curvature matching the curvature of the contact lenses (L); and
secondly, at least one lid (2) designed to cover said cavity (10) comprising a wedge element (21) having bactericidal action and whose outside face (21a) has curvature complementary to the curvature of the support element (11) so that it protects and/or decontaminates a contact lens (L) by holding it stationary in said cavity (10) between said support element (11) and said wedge element (21), **characterised in that** the inside face of the cavity (10) is provided with at least one socket (20) which is suitable for being engaged in guided manner into said cavity (10), with the wedge element (21) being received in the socket (20).

2. A device according to claim 1, **characterised in that** said support element (11) and said wedge element (21) are made of a porous material containing a non-migrant anti-bacterial agent.

3. A device according to any preceding claim, **characterised in that** said wedge element (21) is radially clamped in the socket (20).

4. A device according to any preceding claim, **characterised in that** the edge of said cavity (10) is provided with a collar (12) whose inside wall is provided with a bevel (122) serving to guide the socket (20).

5. A device according to any preceding claim, **characterised in that** the end wall of the cavity (10) is provided with a bore (100) of small diameter and in which the support element (11) is radially clamped.

6. A device according to any preceding claim, **characterised in that** the peripheral edge of the socket (20) is provided with a guide bevel (22).

7. A device according to any preceding claim, **characterised in that** the outside face (11a) of the support element (11) is concave.

8. A device according to any preceding claim, **characterised in that** the outside face (21a) of the wedge element (21) projects beyond the peripheral edge of the socket (20).

9. A device according to any preceding claim, **characterised in that** the support element (11) and the wedge element (21) are removable cylinders having the same diameter.

10. A device according to any preceding claim, **characterised in that** the respective dimensions of the cavity (10) and of the socket (20) are determined so that the outside wall of said socket comes into sliding contact with the inside wall of said cavity.

11. A device according to any preceding claim, **characterised in that** the housing (1) is provided with two cavities (10a, 10b).

12. A device according to claim 11, **characterised in that** it has a single lid (2) which is provided with two sockets (20a, 20b) designed to be inserted into said cavities, and which is assembled to the housing (1) by interfitting with peripheral radial clamping, thereby forming an accurately circularly symmetrical cylinder.

13. A device according to claim 11, **characterised in that** it has two lids (2a, 2b), each of which is provided with a respective socket (20a, 20b).

14. A device according to any preceding claim, **characterised in that** the housing (1) and the lid (2) are made of a plastics material containing a non-migrant anti-bacterial agent.

## Patentansprüche

1. Vorrichtung zur Konditionierung und bakteriziden Behandlung von Kontaktlinsen (L), die umfasst
- einerseits ein Gehäuse (1), das mit mindestens einem Hohlraum (10) versehen ist, in dem ein Trägerelement (11) mit bakterizider Wirkung angeordnet ist, dessen äußere Oberfläche (11a) eine Krümmung aufweist, die derjenigen der Linsen (L) entspricht, und
- andererseits mindestens eine Verschlusskappe (2) zum Abdecken des Hohlraums (10), die umfasst ein Positionierungselement (21) mit bakterizider Wirkung, dessen äußere Oberfläche (21a) eine Krümmung aufweist, die komplementär zu derjenigen des Trägerelements (11) ist, um den Schutz und/oder die Dekontamination einer Linse (L) zu gewährleisten, die innerhalb des Hohlraums (10) zwischen dem genannten Trägerelement (11) und dem genannten Positionierungselement (21) immobilisiert ist,
**dadurch gekennzeichnet, dass** die innere Oberfläche des Hohlraums (10) mit mindestens einer Hülse (20) ausgestattet ist, die mittels einer Führung in dem Hohlraum (10) angeordnet sein kann, wobei das Positionierungselement (21) in der Hülse (20) angeordnet ist.

2. Vorrichtung nach Anspruch 1, **dadurch gekennzeichnet, dass** das Trägerelement (11) und das Positioninierungselement (21) aus einem porösen Material hergestellt sind, das ein nicht wanderndes antibakterielles Agens enthält.

3. Vorrichtung nach einem der vorhergehenden Ansprüche, **dadurch gekennzeichnet, dass** das Positionierungselement (21) radial in die Hülse (20) hineingepresst ist.

4. Vorrichtung nach einem der vorhergehenden Ansprüche, **dadurch gekennzeichnet, dass** der Rand des Hohlraums (10) mit einem Kragen (12) ausgestattet ist, dessen Innenwand eine Abschrägung (122) aufweist, welche die Führung der Hülse (20) gewährleisten soll.

5. Vorrichtung nach einem der vorhergehenden Ansprüche, **dadurch gekennzeichnet, dass** der Boden des Hohlraums (10) eine Bohrung (100) mit einem engen Durchmesser aufweist, in die das Trägerelement (11) radial hineingepresst ist.

6. Vorrichtung nach einem der vorhergehenden Ansprüche, **dadurch gekennzeichnet, dass** der Umfangsrand der Hülse (20) eine Führungs-Abschrägung (22) aufweist.

7. Vorrichtung nach einem der vorhergehenden Ansprüche, **dadurch gekennzeichnet, dass** die äußere Oberfläche (11a) des Trägerelements (11) konkav ist.

8. Vorrichtung nach einem der vorhergehenden Ansprüche, **dadurch gekennzeichnet, dass** die äußere Oberfläche (21a) des Positionierungselements (21) über den Umfangsrand der Hülse (20) vorsteht.

9. Vorrichtung nach einem der vorhergehenden Ansprüche, **dadurch gekennzeichnet, dass** das Trägerelement (11) und das Positionierungselement (21) herausnehmbare Zylinder mit dem gleichen Durchmesser sind.

10. Vorrichtung nach einem der vorhergehenden Ansprüche, **dadurch gekennzeichnet, dass** die jeweiligen Dimensionen des Hohlraums (10) und der Hülse (20) so festgelegt sind, dass die äußere Wand der Hülse mit der inneren Wand des Hohlraums in Gleitkontakt steht.

11. Vorrichtung nach einem der vorhergehenden Ansprüche, **dadurch gekennzeichnet, dass** das Gehäuse (1) zwei Hohlräume (10a,10b) umfasst.

12. Vorrichtung nach Anspruch 11, **dadurch gekennzeichnet, dass** sie eine einzige Verschlusskappe (2) aufweist, die mit zwei Hülsen (20a,20b) ausgestattet ist, die in die Hohlräume einzuführen sind und die mit dem Gehäuse-Einsteckende (1) unter radialem Umfangsdruck unter Bildung eines vollständigen Zylinders eingesetzt sind.

13. Vorrichtung nach Anspruch 11, **dadurch gekennzeichnet, dass** sie zwei Verschlusskappen (2a,2b) umfasst, die jeweils mit einer Hülse (20a,20b) ausgestattet sind.

14. Vorrichtung nach einem der vorhergehenden Ansprüche, **dadurch gekennzeichnet, dass** das Gehäuse (1) und die Verschlusskappe (2) aus einem Kunststoffmaterial hergestellt sind, das ein nicht wandemdes antibakterielles Agens enthält.
